# EUROPÄISCHE PATENTSCHRIFT

(11) **EP 0 537 606 B1**
(45) Veröffentlichungstag und Bekanntmachung des Hinweises auf die Patenterteilung: **29.01.1997**
(21) Anmeldenummer: 92117085.8
(22) Anmeldetag: 07.10.1992
(51) Int. Cl.: C07D 207/26, C07D 313/04

(54) **Verfahren zur Herstellung von 5-Hydroxyethylpyrrolidonen**
Process for the preparation of 5-hydroxyethylpyrrolidones
Procédé pour la préparation de 5-hydroxyéthylpyrrolidones

(30) Priorität: 17.10.1991 DE 4134303
(43) Veröffentlichungstag der Anmeldung: 21.04.1993
(73) Patentinhaber: BASF Aktiengesellschaft, 67063 Ludwigshafen (DE)
(72) Erfinder: Fischer, Rolf, Dr., W-6900 Heidelberg (DE)

(56) Entgegenhaltungen:
- EP-A- 0 270 093
- GB-A- 1 312 463
- US-A- 3 875 184
- PATENT ABSTRACTS OF JAPAN vol. 13, no. 487 (C-649)(3835) 6. November 1989 & JP-A-1190667
- CHEMICAL ABSTRACTS, vol. 113, no. 25, 17. Dezember 1990, Columbus, Ohio, US; abstract no. 231208s, N. VAIDEANU ET AL. & RO-A-96953
- CHEMICAL ABSTRACTS, vol. 92, no. 24, 16. Juni 1980, Columbus, Ohio, US; abstract no. 199096p, S. CIBOROWSKI ET AL. & CHEM. STOSOW. Bd. 23, Nr. 4, 1979, Seiten 467-76
- JOURNAL OF ORGANIC CHEMISTRY Bd. 51, Nr. 1, 1986, EASTON US Seiten 40 - 45 J. D'ANGELO ET AL. ''a short, efficient, highly selective synthesis of (1R,3S)- cis-Chrysanthemic Acid...etc.''

## Beschreibung

Die vorliegende Erfindung betrifft ein Verfahren zur Herstellung von 5-Hydroxyethylpyrrolidonen, aus Caprolactonen mit Ammoniak oder primären Aminen.

Es ist aus EP-A-293 740 bekannt, daß man 5-Acetoxyethylpyrrolidon durch Umsetzung von Pyrrolidon mit Vinylacetat in Gegenwart von Di-tert.-butylperoxid erhalten kann.

Ferner ist aus C.H. Hassall, Org. React, Band 9, Seite 73 ff (1957) bekannt, Ketone mit Hilfe von Persäuren zu Estern zu oxidieren (Baeyer-Villiger-Oxidation). Setzt man cyclische Ketone ein, so erhält man dabei Lactone, aus Cyclohexanon z.B. Caprolacton.

Es ist weiterhin aus J. Org. Chem. 51, Seiten 40 bis 45 (1986) bekannt, daß man durch Umsetzung von 2,2,5,5-Tetra-methyl-4-hydroxycyclohexanon mit m-Chlorperbenzoesäure das γ-Lacton der 4,6-Dihydroxy- 3,3,6-trimethylheptansäure erhält.

Der vorliegenden Erfindung lag die Aufgabe zugrunde, ein neues und günstiges Verfahren zur Herstellung von 5-Hydroxyethylpyrrolidonen zu finden.

Demgemäß wurde ein neues und verbessertes Verfahren zur Herstellung von 5-Hydroxyethylpyrrolidonen der allgemeinen Formel I 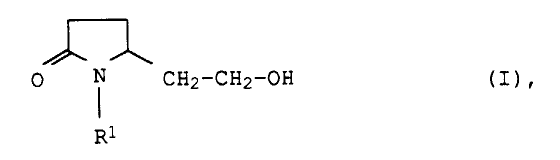 in der
- R¹: Wasserstoff, C₁- bis C₁₂-Alkyl, C₃- bis C₈-Cycloalkyl, Aryl oder C₇- bis C₁₂-Aralkyl
bedeutet, welches dadurch gekennzeichnet ist, daß man ein Caprolacton der allgemeinen Formel II 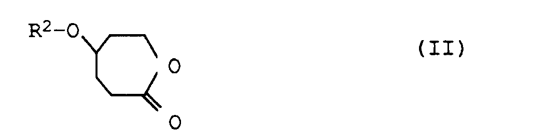 in der
- R²: C₁- bis C₁₂-Alkyl, 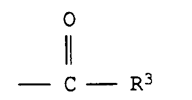 und
- R³: C₁- bis C₈-Alkyl und Phenyl
bedeuten, mit Ammoniak oder primären Aminen der allgemeinen Formel III

R¹-NH₂ (III)

in der R¹ die obengenannten Bedeutungen hat, bei Temperaturen von 150 bis 450°C und Drücken von 10 bis 350 bar umsetzt, sowie die Herstellung der Caprolactone II aus den Cyclohexanonen IV.

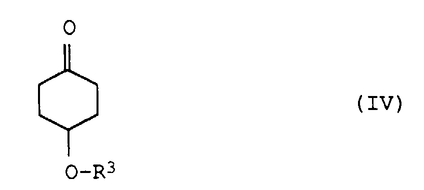

Das erfindungsgemäße Verfahren zur Herstellung von 5-Hydroxyethylpyrrolidonen I kann wie folgt durchgeführt werden:

Das Caprolacton II kann mit Ammoniak oder einem primären Amin III bei erhöhten Temperaturen und erhöhten Drücken in der Gasphase, insbesondere aber in der Flüssigphase diskontinuierlich oder bevorzugt kontinuierlich umgesetzt werden.

Die Umsetzung kann beispielsweise so durchgeführt werden, daß man ein Gemisch aus Ausgangsverbindung II und Ammoniak oder primärem Amin, gegebenenfalls in Gegenwart von Wasser oder einem unter den Reaktionsbedingungen inerten Lösungsmittel in einem Autoklaven so lange unter dem sich einstellenden Eigendruck des Systems erhitzt, bis die Umsetzung vollständig abgelaufen ist. Anschließend wird abgekühlt und entspannt. Die Aufarbeitung der Reaktionsausträge kann in an sich bekannter Weise, z.B. durch Destillation oder Kristallisation erfolgen.

Ammoniak und primäre Amine können in reiner Form oder, als wäßriger Ammoniak und soweit die Amine ausreichend wasserlöslich sind, als wäßrige Lösungen eingesetzt werden.

Als primäre Amine kommen z.B. Alkylamine, Cycloalkylamine, weiterhin Arylamine und Aralkylamine in Frage. Beispiele für derartige primäre Amine sind Methylamin, n-Pentylamin, n-Dodecylamin, Cyclopentylamin, Cyclohexylamin, Anilin oder Benzylamin.

Es ist auch möglich, in Gegenwart von unter den Reaktionsbedingungen inerten Lösungsmitteln zu arbeiten. Als Lösungsmittel hierfür können z. B. Wasser, Alkohole, Ether, Kohlenwasserstoffe, aromatische Verbindungen, chlorierte Kohlenwasserstoffe oder deren mischbare Gemische verwendet werden.

Die Umsetzung kann bei Temperaturen von 150 bis 400°C, insbesondere 250 bis 350°C und Drücken von 10 bis 350 bar, bevorzugt 50 bis 250 bar durchgeführt werden. Gearbeitet wird im allgemeinen in Druckbehältern wie Autoklaven unter dem Eigendruck des Systems. Die Reaktionszeiten betragen in der Regel 0,5 bis 5 Stunden. Das Molverhältnis von Ammoniak oder Amin zu Caprolacton II beträgt 0,5 : 1 bis 50 : 1, insbesondere 1 : 1 bis 20 : 1.

Die Caprolactone II lassen sich z.B. durch Umsetzung von in 4-Stellung entsprechend substituierten Cyclohexanonen mit Percarbonsäuren (Baeyer-Villiger-Oxidation) herstellen.

Die Umsetzung läßt sich wie folgt durchführen:
Das in 4-Stellung durch Alkoxy- oder Acyloxygruppen substituierte Cyclohexanon wird in der Flüssigphase mit einer Percarbonsäure umgesetzt, die in einem unter den Reaktionsbedingungen inerten Lösungsmittel gelöst ist.

Die Umsetzung kann beispielsweise so durchgeführt werden, daß man zum Cyclohexanon IV eine Lösung der Persäure V bei der jeweiligen Reaktionstemperatur in dem Maße zufügt, wie sie abreagiert. Das Cyclohexanon IV kann ebenso wie die Persäure V in einem Lösungsmittel zugeführt werden. Dabei ist es zweckmäßig, aber nicht zwingend, für das Cyclohexanon IV und die Persäure V das gleiche Lösungsmittel zu verwenden.

Der Verlauf der Reaktion kann z.B. durch Titration der jeweils verwendeten Persäure verfolgt werden. Die Umsetzung wird im allgemeinen so lange fortgeführt, bis das Cyclohexanon IV vollständig abreagiert ist. Spuren von Persäure-/Peroxidverbindungen können z.B. durch nachträgliches Erhitzen oder durch Zusatz von Katalysatoren, die die Zersetzung von Persäure-/Peroxidverbindungen katalysieren, zerstört werden. Die Aufarbeitung der Reaktionsgemische kann in an sich bekannter Weise, z.B. durch Destillation oder Kristallisation, erfolgen.

Als Persäuren V kommen alle üblichen Persäuren in Betracht wie z.B. Perameisensäure, Peressigsäure, Perpropionsäure, Trifluorperessigsäure, Monoperphthalsäure, Perbenzoesäure, m-Chlorperbenzoesäure, Permaleinsäure.

Als Lösungsmittel eignen sich Carbonsäuren z.B. C₁- bis C₈-Carbonsäuren, bevorzugt C₁- bis C₄-Carbonsäuren wie Ameisensäure, Essigsäure, Propionsäure, chlorierte Kohlenwasserstoffe wie Methylenchlorid, Chloroform oder 1,2-Dichlorethan, aromatische Kohlenwasserstoffe wie Benzol, Toluol oder Xylole. Im allgemeinen sollten die Lösungsmittel unter den Reaktionsbedingungen stabil sein.

Es kann auch Wasserstoffperoxid, gelöst in einer Carbonsäure, gegebenenfalls in Gegenwart von sauren Katalysatoren wie z. B. Schwefelsäure oder stark sauren Ionentauschern verwendet werden. Es ist weiterhin möglich, die Cyclohexanone IV mit Wasserstoffperoxid in Gegenwart von zeolithischen Katalysatoren umzusetzen (US-A-4,870,192).

Die Umsetzung kann bei Temperaturen von (-20) bis 150°C, insbesondere von 20 bis 120 °C und Drücken von 0,1 bis 2 bar, bevorzugt von 0,5 bis 1,3 bar, im allgemeinen bei Atmosphärendruck (Normaldruck) durchgeführt werden.

Die Umsetzung kann in flüssiger Phase diskontinuierlich oder kontinuierlich durchgeführt werden.

Das Molverhältnis von der Persäure V zum Cyclohexanon IV beträgt 1 : 1 bis 2 : 1, insbesondere 1 : 1, 1,5 : 1.

Die Cyclohexanone IV lassen sich beispielsweise durch Hydrierung von Hydrochinon und Hydrochinon-Derivaten in Gegenwart von Palladium-Katalysatoren herstellen.

Die Substituenten R¹, R² und R³ in den Verbindungen I, II, III, IV und V haben folgende Bedeutungen:
R¹
- Wasserstoff,
- C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, tert.-Amyl, n-Hexyl, iso-Hexyl, n-Heptyl, iso-Heptyl, n-Octyl und iso-Octyl, besonders bevorzugt C1- bis C4-Alkyl wie Methyl, Ethyl, n-Propyl, iso-Propyl, n-Butyl, iso-Butyl, sec.-Butyl und tert.-Butyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- C₇- bis C₁₂-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl,1-Phenethyl und 2-Phenethyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
R²
- C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, tert.-Amyl, n-Hexyl, iso-Hexyl, n-Heptyl, iso-Heptyl, n-Octyl und iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl, 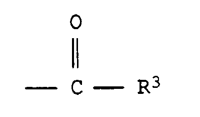
R³
- C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, tert.-Amyl, n-Hexyl, iso-Hexyl, n-Heptyl, iso-Heptyl, n-Octyl und iso-Octyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
- Phenyl.

Die nach dem erfindungsgemäßen Verfahren herstellbaren 5-Hydroxyethylpyrrolidone I, die Caprolactone II und die Cyclohexanone IV stellen wichtige Zwischenprodukte für die Synthese von Pharmavorprodukten wie z. B. 5-Vinylpyrrolidon (US-A-4 235 778, US-A-3 959 356) dar.

Durch Abspaltung von Wasser, oder nach Acetylierung durch Abspaltung von Essigsäure, erhält man 5-Vinylpyrrolidon, das ein Zwischenprodukt für die Synthese des Antiepileptikums Vinyl-Gaba darstellt.

### Beispiele

### Beispiel 1

### Herstellung von 4-Acetoxycaprolacton

50 g 4-Acetoxycyclohexanon, gelöst in 150 ml Chloroform, wurden bei 40°C innerhalb von 40 Minuten unter Rühren mit 136 g m-Chlorperbenzoesäure (55 %ig), gelöst in 500 ml Chloroform, versetzt. Das Reaktionsgemisch wurde 2 Stunden bei 40°C nachgerührt. Nach dem Abkühlen auf Raumtemperatur wurde der ausgefallene Feststoff (m-Chlorbenzoesäure) abgesaugt. Die Chloroform-Phase wurde zweimal mit 20 %iger wäßriger Sodalösung ausgeschüttelt, über Natriumsulfat getrocknet und am Rotationsverdampfer eingeengt. Aus dem hierbei anfallenden Rückstand wurden durch Kugelrohrdestillation 46,4 g 4-Acetoxycaprolacton (84 %, bezogen auf eingesetztes 4-Acetoxycyclohexanon) gewonnen.

¹³C-NMR-Spektrum (CDCl₃)
δ [ppm] = 21,1 (CH₃), 27,5 (CH₂), 28,4 (CH₂), 33,9 (CH₂), 63,5 (CH₂), 70,0 (CH), 169,9 (C=O), 175,0 (C=O).

¹H-NMR-Spektrum (CDCl₃)
δ [ppm] = 2,0 (2H,m), 2,1 (2H,m), 2,1 (3H,S), 2,5 (1H,m), 2,9 (1H,M), 4,2 (1H,m), 4,45 (1H,m, 5,1 (1H,m).

### Beispiel 2

### Herstellung von 5-Hydroxyethylpyrrolidon

In einem Autoklaven wurden 22,4 g 4-Acetoxycaprolacton zusammen mit 100 g 25 %igem wäßrigem Ammoniak eine Stunde lang bei 330°C gerührt. Nach Abkühlen und Entspannen des Autoklaven wurde der Reaktionsaustrag am Rotationsabdampfer (40°C/30 mbar) eingeengt und durch Kurzwegdestillation gereinigt. Hierbei wurden 10,1 g 5-Hydroxyethylpyrrolidon erhalten (61 %, bezogen auf eingesetztes 4-Acetoxycaprolacton) und NMR-spektroskopisch identifiziert.

### Beispiel 3

### Herstellung von 4-Methoxycaprolacton

In gleicher Weise wie in Beispiel 1 beschrieben, wurden 14,8 g 4-Methoxycyclohexanon mit m-Chlorperbenzoesäure umgesetzt und aufgearbeitet. Dabei wurden 9 g 4-Methoxycaprolacton (n_{D} ²⁰ = 1,4737) (54 %, bezogen auf eingesetztes 4-Methoxycyclohexanon) gewonnen.

¹³C-NMR-Spektrum (CDCl₃)
δ [ppm] = 26,9 (CH₂), 27,8 (CH₂), 33,6 (CH₂), 55,9 (CH₃), 63,4 (CH₂), 75,6 (CH), 176,1 (C=O).

¹H-NMR-Spektrum (CDCl₃)
δ [ppm] = 1,85 (1H,m), 2,0 (3H,m), 2,4 (1H,q), 2,95 (1H,t), 3,35 (3H,s), 3,6 (1H,m), 4,1 (1H,q), 4,5 (1H,t).

### Beispiel 4

### Herstellung von 5-Hydroxyethylpyrrolidon

In gleicher Weise, wie in Beispiel 1 beschrieben, wurden 30 g 4-Methoxycaprolacton zusammen mit 100 g 25 %igem wäßrigem Ammoniak eine Stunde lang bei 330°C gerührt. Nach Abkühlen und Entspannen des Autoklaven und destillativer Aufarbeitung wurden 9,1 g 5-Hydroxyethylpyrrolidon erhalten (34 %, bezogen auf eingesetztes 4-Methoxycaprolacton) und NMR-spektroskopisch identifiziert.

## Patentansprüche

1. Verfahren zur Herstellung von 5-Hydroxyethylpyrrolidonen der allgemeinen Formel I 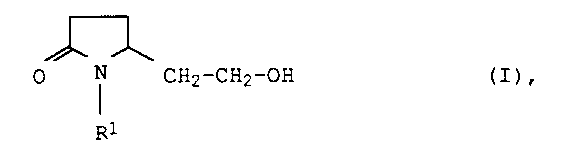 in der
R¹ Wasserstoff, C₁- bis C₁₂-Alkyl, C₃- bis C₈-Cycloalkyl, Aryl oder C₇- bis C₁₂-Aralkyl bedeutet,
dadurch gekennzeichnet, daß man
ein Caprolacton der allgemeinen Formel II 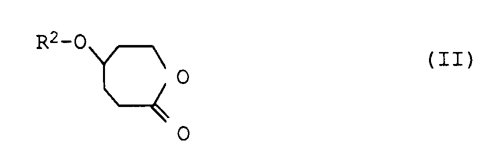 in der
R² C₁- bis C₁₂-Alkyl, 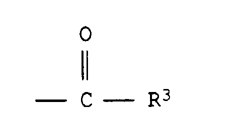 und
R³ C₁- bis C₈-Alkyl und Phenyl
bedeuten, mit Ammoniak oder primären Aminen der allgemeinen Formel III
R¹-NH₂ (III)
in der R¹ die obengenannten Bedeutungen hat, bei Temperaturen von 150 bis 450°C und Drücken von 10 bis 350 bar umsetzt.

2. Verfahren zur Herstellung von 5-Hydroxyethylpyrrolidonen der allgemeinen Formel I nach Anspruch 1, dadurch gekennzeichnet, daß man die Caprolactone der allgemeinen Formel II durch Umsetzung von Cyclohexanonen der allgemeinen Formel IV 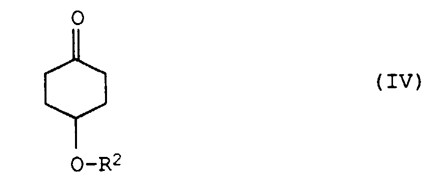 in der R² die obengenannten Bedeutungen hat, mit einer Persäure der allgemeinen Formel V 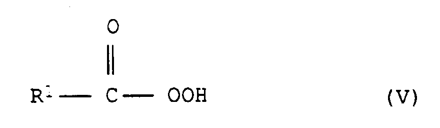 in der R¹ die obengenannten Bedeutungen hat, bei Temperaturen von (-20) bis +150°C und Drücken von 0,1 bis 2 bar erhält.

3. Caprolactone der allgemeinen Formel II nach Anspruch 1, in der R² 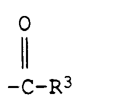 und R³ C₁- bis C₈-Alkyl und Phenyl bedeutet.

## Claims

1. A process for preparing a 5-hydroxyethylpyrrolidone of the general formula I 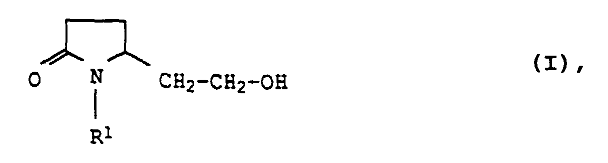 where
R¹ is hydrogen, C₁- to C₁₂-alkyl, C₃- to C₈-cycloalkyl, aryl or C₇- to C₁₂-aralkyl,
which comprises reacting
a caprolactone of the general formula II 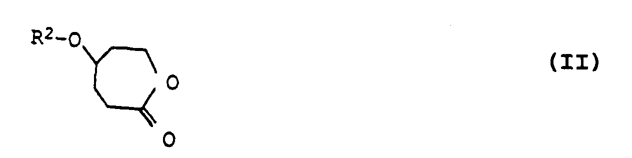 where
R² is C₁- to C₁₂-alkyl or 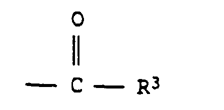
R³ is C₁- to C₈-alkyl or phenyl,
with ammonia or a primary amine of the general formula III
R¹-NH₂ (III)
where R¹ has the abovementioned meanings, at from 150 to 450°C and 10 to 350 bar.

2. A process for preparing a 5-hydroxyethylpyrrolidone of the general formula I as claimed in claim 1, wherein the caprolactone of the general formula II is obtained by reacting a cyclohexanone of the general formula IV 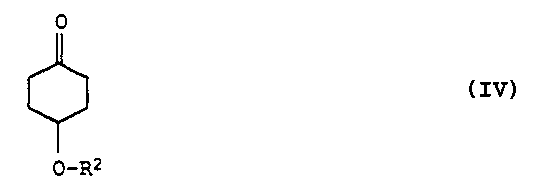 in which R² has the abovementioned meanings, with a peracid of the general formula V 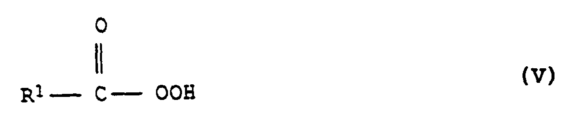 in which R¹ has the abovementioned meanings, at from -20 to +150°C and at from 0.1 to 2 bar.

3. A caprolactone of the general formula II as claimed in claim 1, where R² is 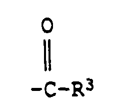 and R³ is C₁- to C₈-alkyl or phenyl.

## Revendications

1. Procédé de préparation de 5-hydroxyéthylpyrrolidones de la formule I 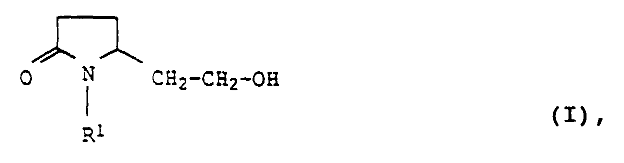 dans laquelle
R¹ représente un atome d'hydrogène, un radical alkyle en C₁ à C₁₂, cycloalkyle en C₃ à C₈, aryle ou aralkyle en C₇ à C₁₂,
caractérisé en ce que l'on fait réagir
une caprolactone de la formule générale II 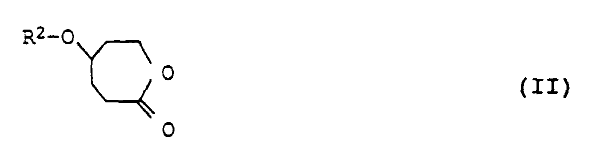 dans laquelle
R² représente un radical alkyle en C₁ à C₁₂, 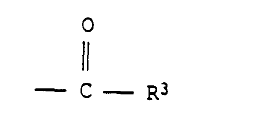 et
R³ représente un radical alkyle en C₁ à C₈ et phényle,
avec de l'ammoniac ou des amines primaires de la formule générale III
R¹-NH₂ (III)
dans laquelle R¹ possède les significations qui lui ont été attribuées ci-dessus, à des températures de 150 à 450°C et sous des pressions de 10 à 350 bars.

2. Procédé de préparation de 5-hydroxyéthylpyrrolidones de la formule générale I suivant la revendication 1, caractérisé en ce que l'on obtient les caprolactones de la formule générale II par la réaction de cyclohexanones de la formule générale IV 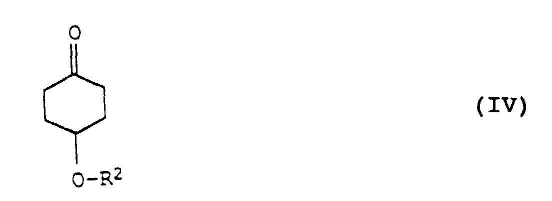 dans laquelle R² possède les significations qui lui ont été précédemment attribuées, avec un peracide de la formule V 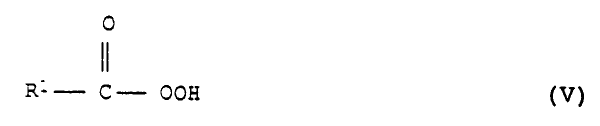 dans laquelle R¹ possède les significations qui lui ont été attribuées ci-dessus, à des températures de (-20°C) à +150°C et sous des pressions de 0,1 à 2 bars.

3. Caprolactones de la formule générale II suivant la revendication 1, dans laquelle R² représente un radical 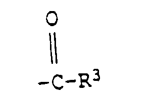 et R³ représente un radical alkyle en C₁ à C₈ et phényle.
